(19) Europäisches Patentamt
European Patent Office
Office européen des brevets

(11) Veröffentlichungsnummer: **0 199 972 B1**

(12) # EUROPÄISCHE PATENTSCHRIFT

(45) Veröffentlichungstag der Patentschrift: **13.09.89**

(51) Int. Cl.[4]: **C07C 59/245**, C12P 7/42, C12N 1/28 // (C12P7/42, C12R1:74)

(21) Anmeldenummer: **86103828.9**

(22) Anmeldetag: **20.03.86**

(54) **3-Hydroxydicarbonsäuren und ein Verfahren zu ihrer Herstellung.**

(30) Priorität: **23.04.85 DE 3514550**

(43) Veröffentlichungstag der Anmeldung: **10.12.86 Patentblatt 86/45**

(45) Bekanntmachung des Hinweises auf die Patenterteilung: **13.09.89 Patentblatt 89/37**

(84) Benannte Vertragsstaaten: **AT CH DE FR GB IT LI NL**

(56) Entgegenhaltungen:
**GB-A- 1 196 596**
**GB-A- 2 031 885**
**US-A- 2 831 891**

(73) Patentinhaber: **HÜLS AKTIENGESELLSCHAFT, Patentabteilung / PB 15 - Postfach 13 20, D-4370 Marl 1(DE)**

(72) Erfinder: **Hill, Frank Friedrich, Dr., Schlesienstrasse 23, D-4020 Mettmann(DE)**

**EP 0 199 972 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft unverzweigte, gesättigte 3-Hydroxydicarbonsäuren der Formel

$$HOOC-CH_2-\underset{|}{\overset{OH}{CH}}-(CH_2)_n-COOH \quad (I),$$

wobei n eine ganze Zahl von 9 bis 14 ist, und ein mikrobiologisches Verfahren zur Herstellung der Verbindung der Formel I, wobei n eine ganze Zahl von 8 bis 14 ist.

Derartige Verbindungen eignen sich als Ausgangsstoffe für synthetische Polymere, Tenside, Emulgatoren und Moschus-Riechstoffe, die man durch Cyclisierungsreaktionen erhalten kann.

Kurzkettige 3-Hydroxydicarbonsäuren sind bekannt. So kommt die Äpfelsäure (für n = 0 in Formel I) in Früchten vor. 3-Hydroxyadipinsäure (für n = 2 in Formel I) wurde von H. Arakawa et al. [Liebigs Ann. Chem. 728 (1969), 152] auf chemischem Wege hergestellt. Ein mikrobiologisches Herstellverfahren ist aber für diese Verbindung nicht bekannt. 3-Hydroxydodecandisäure (für n = 8 in Formel I) wird bei der chemischen Synthese von Traumatinsäure als Zwischenprodukt erwähnt [P.H.M. Schreurs et al., Rec. Trav. Chim. Pays-Bas 90 (1971), 1331]. Ein mikrobiologisches Verfahren zur präparativen Herstellung von 3-Hydroxydodecandisäure gibt es aber noch nicht.

Nach GB-A 2 031 885 können alpha-Olefine mit Hilfe eines Stammes von Candida tropicalis zu Dihydroxycarbonsäuren fermentiert werden. Dabei werden auch 2-Hydroxydicarbonsäuren als Nebenprodukte gebildet.

Unverzweigte, gesättigte 3-Hydroxydicarbonsäuren mit insgesamt 13 bis 18 C-Atomen sind nicht bekannt.

Der Erfindung liegt die Aufgabe zugrunde, bisher nicht bekannte, unverzweigte, gesättigte 3-Hydroxydicarbonsäuren mit 13 bis 18 C-Atomen bereitzustellen und ein Verfahren zur Herstellung von unverzweigten, gesättigten 3-Hydroxydicarbonsäuren mit 12 bis 18 C-Atomen anzugeben.

Diese Aufgabe wird erfindungsgemäß dadurch gelöst, daß n-Alkane mit 12 bis 18 C-Atomen durch mikrobielle Transformation mit Hilfe der alkanabbaudefekten Mutante Candida tropicalis DSM 3152 in unverzweigte, gesättigte 3-Hydroxydicarbonsäuren mit 12 bis 18 C-Atomen übergeführt werden.

Bei der mikrobiellen Transformation wird eine Hefemutante benötigt, die sich aus dem Hefestamm Candida tropicalis (Wildstamm) gewinnen läßt. Während der Wildstamm in einem Nährmedium aus Mineralsalzen und n-Alkanen mit 12 bis 18 C-Atomen als einziger Kohlenstoffquelle wächst, ist diese Eigenschaft in der Mutante Candida tropicalis DSM 3152 völlig aufgehoben. Candida tropicalis DSM 3152 hat folgende Eigenschaften:

– Aerobes Wachstum in Glucose-Hefeextrakt-Pepton-Medium in Form ovaler Zellen mit einem Durchmesser von 2 bis 6 µm.

– Aerobes Wachstum in einer mineralischen Nährlösung mit Zusatz von Vitaminen und den Kohlenstoffquellen Glycerin, Ethanol, Bernsteinsäure oder D-Glucose. Gutes Wachstum nach Zusatz von D-Biotin.

– Kein Wachstum in einer mineralischen Nährlösung mit Zusatz von n-Alkanen mit 12 bis 18 C-Atomen (auch nicht nach Zusatz von Emulgatoren) und keine Fähigkeit mehr, diese Alkane abzubauen. Deshalb wird dieser Mikroorganismus als «alkanabbaudefekte» Mutante bezeichnet.

– Unter guten Wachstumsbedingungen werden n-Alkane mit 12 bis 18 C-Atomen in 3-Hydroxydicarbonsäuren mit der gleichen Anzahl an C-Atomen übergeführt.

**Tabelle 1: Unterschiede zwischen Wildtyp und Mutante**

| | Wachstum auf Mineralsalz und | | | | | | |
|---|---|---|---|---|---|---|---|
| | Glycerin | Glucose | Dodecan | Tridecan | Tetradecan | Pentadecan | Hexadecan |
| Candida tropicalis (Wildtyp) | ++ | +++ | ++ | ++ | ++ | ++ | +(+) |
| Mutante DSM 3152 | ++ | +++ | – | – | – | – | – |

Die guten Wachstumsbedingungen erfordern ein Nährmedium, das enthalten kann:

– Anorganische Salze aus Kationen wie Ammonium, Kalium, Natrium, Calcium, Magnesium, Mangan, Eisen und den Anionen Phosphat, Nitrat, Chlorid, Suflat und Carbonat.

– Organische Nährstoffe einfacher Zusammensetzung wie Ethanol, Glycerin, Essigsäure und Glucose.

– Organische Nährstoffe komplexer Zusammensetzung wie Hefeextrakt, Maisquellwasser und Peptone aus pflanzlichen und/oder tierischen Proteinen.

Zu den guten Wachstumsbedingungen gehören außerdem:

– Temperatur von 20 bis 40°C

– pH-Wert von 3 bis 8

– Belüftung der Kulturbrühe aus Hefe und Nährmedium.

Das umzusetzende n-Alkan kann der Kulturbrühe auf einmal, in einzelnen Portionen oder auch kontinuierlich zugegeben werden. In gleicher Weise können auch Emulgatoren, durch die die Umsetzung der n-Alkane beschleunigt wird, zugesetzt werden.

Da während der Fermentation durch die Bildung der Säuren der pH-Wert des Ansatzes fällt, müssen Basen zudosiert werden, um den pH-Wert im gewünschten Bereich zu halten. Gebräuchliche Basen sind Ammoniak, Natronlauge, Kalilauge oder Calciumhydroxid.

Der für die Umwandlung der n-Alkane in 3-Hydroxydicarbonsäuren erforderliche Sauerstoff wird durch Belüften der Kulturbrühe mit Luft oder mit Sauerstoff angereicherter Luft eingeführt.

Nach beendeter Fermentation kann die Abtrennung und Reinigung der 3-Hydroxydicarbonsäuren nach üblichen Verfahren erfolgen. Geeignet sind Fällung, Extraktion, Ionenaustauschchromatographie und Elektrodialyse.

Ein bevorzugter Weg zur Reindarstellung der 3-Hydroxydicarbonsäuren besteht aus folgenden Schritten:

1. Einstellung der Kulturbrühe auf pH > 8.
2. Durch Zentrifugieren werden Hefebiomasse und nicht umgesetzte n-Alkane abgetrennt.
3. Durch Ansäuern der Lösung auf pH < 5 werden die 3-Hydroxydicarbonsäuren und weitere Carboxylgruppen enthaltende Produkte ausgefällt.
4. Die ausgefällten Produkte werden abgetrennt, gewaschen, getrocknet und dann mit Methanol verestert.
5. Chromatographische Abtrennung und Reinigung der 3-Hydroxydicarbonsäuredimethylester.
6. Alkalische Verseifung zu den 3-Hydroxydicarbonsäuren.

Damit sind die bisher nicht bekannten 3-Hydroxydicarbonsäuren mit 13 bis 18 C-Atomen zugänglich geworden. Diese Produkte sind frei von physiologisch bedenklichen Verunreinigungen.

Das erfindungsgemäße Verfahren ermöglicht es, aus n-Alkanen auf einfachem Wege unter milden Bedingungen direkt 3-Hydroxydicarbonsäuren mit 12 bis 18 C-Atomen herzustellen.

Die folgenden Beispiele sollen die Erfindung erläutern.

Beispiel 1

Es wird eine Nährlösung mit Substrat angesetzt, die pro Liter

| | |
|---|---|
| 3 g | $NH_4NO_3$ |
| 3 g | $(NH_4)_2SO_4$ |
| 1,5 g | $KH_2PO_4$ |
| 1 g | $K_2HPO_4$ |
| 6 g | Maisquellwasser |
| 0,2 g | KCl |
| 4 mg | $FeSO_4 \cdot 7H_2O$ |
| 0,4 mg | $ZnSO_4 \cdot 7H_2O$ |
| 1 mg | $MnSO_4 \cdot H_2O$ |
| 10 g | Glycerin |
| 2 g | Emulgator (BRIJ $35^R$) |
| 6 g | n-Dodecan |

enthält.

In 500 ml dieser Lösung wird der Hefestamm DSM 3152 geimpft und dann 3 Tage bei 30°C aerob gezüchtet. Mit den 500 ml der so gebildeten Vorkultur werden anschließend in einem 10-l-Fermenter 5 l Lösung der obengenannten Zusammensetzung beimpft. Der 5,5-l-Ansatz wird bei 30°C mit 600 Upm gerührt und mit 1 Liter Luft pro Liter Reaktorvolumen und Minute (1 vvm) belüftet.

Der pH-Wert wird durch Zugabe von 8 N NaOH auf pH 6,0 gehalten. Nach 20 Stunden wird mit der Zufütterung von 1 l n-Dodecan und 600 ml 10%iger Ammoniumacetat-Lösung begonnen, wobei pro Stunde 20 ml n-Dodecan und 12 ml Ammoniumacetat-Lösung in den Fermenter dosiert werden. Gleichzeitig wird der pH-Wert des Reaktionsansatzes von pH 6,0 auf pH 7,5 angehoben. Nach 90 Stunden Laufzeit wird die Fermentation beendet und der Ansatz aufgearbeitet.

Dazu wird die Kulturbrühe auf pH 9 eingestellt und die Biomasse und restliches n-Dodecan durch Zentrifugation abgetrennt. Die gebildeten langkettigen Disäuren werden durch Ansäuern mit Salzsäure auf pH 3 ausgefällt und durch Waschen und Trocknen in roher Form als Produktgemisch erhalten. Ausbeute 210 g.

Die gaschromatographische Analyse (Tabelle 2) zeigt, daß in dem Produktgemisch 5% 3-Hydroxydodecandisäure vorhanden sind.

Tabelle 2:

Gaschromatographische Analyse der aus n-Dodecan hergestellten Disäuren

| Identifizierte Säure | Gehalt Masse-% |
|---|---|
| Decandisäure | 2,2 |
| Undecandisäure | 0,3 |
| Dodecandisäure | 91,3 |
| 3-Hydroxydecandisäure | 0,7 |
| 3-Hydroxyundecandisäure | 0,1 |
| 3-Hydroxydodecandisäure | 5,0 |
| | 99,6 |

Beispiel 2

Es wird wie in Beispiel 1 gearbeitet, es wird jedoch n-Tridecan anstelle von n-Dodecan als Substrat verwendet.

Als Produktgemisch gefällter und getrockneter Disäuren werden 550 g erhalten. Die gaschromatographische Analyse zeigt, daß darin 9% 3-Hydroxytridecandisäure enthalten sind (Tabelle 3).

Tabelle 3:

Gaschromatographische Analyse der aus n-Tridecan hergestellten Disäuren

| Identifizierte Säure | Gehalt Masse-% |
|---|---|
| Undecandisäure | 1,0 |
| Dodecandisäure | 0,2 |
| Tridecandisäure | 84,4 |
| 3-Hydroxyundecandisäure | 0,6 |
| 3-Hydroxydodecandisäure | 0,7 |
| 3-Hydroxytridecandisäure | 9,3 |
| | 96,2 |

Beispiel 3

Es wird wie in Beispiel 1 gearbeitet, es wird jedoch n-Tetradecan anstelle von n-Dodecan als Substrat verwendet.

Als Produktgemisch gefällter und getrockneter Disäuren werden 430 g erhalten. Die gaschromatographische Analyse zeigt, daß darin 34% 3-Hydroxytetradecandisäure enthalten sind (Tabelle 4).

Tabelle 4:

Gaschromatographische Analyse der aus n-Tetradecan hergestellten Disäuren

| Identifizierte Säure | Gehalt Masse-% |
|---|---|
| Dodecandisäure | 2,2 |
| Tridecandisäure | 0,3 |
| Tetradecandisäure | 47,8 |
| 3-Hydroxydodecandisäure | 4,8 |
| 3-Hydroxytridecandisäure | 1,0 |
| 3-Hydroxytetradecandisäure | 34,0 |
| | 90,1 |

Beispiel 4

Es wird wie in Beispiel 1 gearbeitet, es wird jedoch n-Pentadecan anstelle von n-Dodecan als Substrat verwendet.

Als Produktgemisch gefällter und getrockneter Disäuren werden 390 g erhalten. Die gaschromatographische Analyse zeigt, daß darin 30% 3-Hydroxypentadecandisäure enthalten sind (Tabelle 5).

Tabelle 5:

Gaschromatographische Analyse der aus n-Pentadecan hergestellten Disäuren

| Identifizierte Säure | Gehalt Masse-% |
|---|---|
| Tridecandisäure | 2,8 |
| Tetradecandisäure | 0,9 |
| Pentadecandisäure | 40,4 |
| 3-Hydroxytridecandisäure | 10,1 |
| 3-Hydroxytetradecandisäure | 1,2 |
| 3-Hydroxypentadecandisäure | 30,4 |
| | 85,8 |

Beispiel 5

Es wird wie in Beispiel 1 gearbeitet, es wird jedoch n-Hexadecan anstelle von n-Dodecan als Substrat verwendet.

Als Produktgemisch gefällter und getrockneter Disäuren werden 330 g erhalten. Die gaschromatographische Analyse zeigt, daß darin 57% 3-Hydroxyhexadecandisäure enthalten sind (Tabelle 6).

Tabelle 6:

Gaschromatographische Analyse der aus n-Hexadecan hergestellten Disäuren

| Identifizierte Säure | Gehalt Masse-% |
|---|---|
| Tetradecandisäure | 0,3 |
| Pentadecandisäure | 0,1 |
| Hexadecandisäure | 7,7 |
| 3-Hydroxytetradecandisäure | 14,9 |
| 3-Hydroxypentadecandisäure | 0,7 |
| 3-Hydroxyhexadecandisäure | 57,0 |
| | 80,7 |

Beispiel 6

Reindarstellung und Identifizierung des 3-Hydroxytetradecandisäuredimethylester.

Das in Beispiel 3 angefallene Gemisch aus Dicarbonsäuren und 3-Hydroxydicarbonsäuren wird mit Methanol unter Katalyse von Schwefelsäure verestert und dann an Kieselgel mit Essigsäureethylester/Hexan (Volumenverhältnis 1:1) als Laufmittel fraktioniert chromatographiert. Dabei wird 3-Hydroxytetradecandisäuredimethylester isoliert und durch folgende Analysen identifiziert:

Elementaranalyse:
63,3% C (berechnet: 63,6%)
10,1% H (berechnet: 9,9%)
26,6% O (berechnet: 26,5%)

Massenspektrum: MG = 302

IR-Spektrum:
3400 $cm^{-1}$ (Hydroxylgruppe)
1740 $cm^{-1}$ (Ester)

$^{13}C$-NMR-Spektrum:
$CDCl_3$ als Lösungsmittel,
TMS als Standard

$$\begin{array}{cccccccccccccccc} & & & & & OH & & & & & & & \\ & & & & & | & & & & & & & \\ CH_3 & OO\,C & - & CH_2 & - & CH & - & CH_2 & \ldots\ldots & CH_2 & - & C\,OO & CH_3 \\ 15 & 1 & & 2 & & 3 & & 4 & \ldots\ldots & 13 & & 14 & 16 \end{array}$$

Tabelle 7:

| Nummer des C-Atoms | Verschiebung ppm |
|---|---|
| | 24,92 |
| | 25,45 |
| 4–13 | 29,10 (unvollständige Auflösung) |
| | 29,20 |
| | 29,37 |
| | 29,46 |
| | 34,06 |
| 2 | 41,14 |
| 15, 16 | 51,36 |
| | 51,64 |
| 3 | 67,93 |
| 1, 14 | 173,25 |
| | 174,11 |

## Patentansprüche

1. 3-Hydroxydicarbonsäuren der Formel

$$HOOC - CH_2 - \overset{\displaystyle OH}{\overset{|}{CH}} - (CH_2)_n - COOH \qquad (I),$$

wobei n eine ganze Zahl von 9 bis 14 ist.

2. Verfahren zur Herstellung von 3-Hydroxydicarbonsäuren der Formel I, wobei n eine ganze Zahl von 8 bis 14 ist, dadurch gekennzeichnet, daß sie aus n-Alkanen mit 12 bis 18 C-Atomen als Substrat durch mikrobielle Transformation mit Hilfe der alkanabbaudefekten Mutante Candida tropicalis DSM 3152 hergestellt werden.

3. Mirkoorganismenstamm DSM 3152 (Candida tropicalis).

## Claims

1. 3-Hydroxy dicarboxylic acids of the formula

$$HOOC - CH_2 - \overset{\displaystyle OH}{\overset{|}{CH}} - (CH_2)_n - COOH \qquad (I),$$

where n is an integer from 9 to 14.

2. Process for the preparation of 3-hydroxy dicarboxylic acids of the formula I, where n is an integer from 8 to 14, characterized in that they are prepared from n-alkanes with 12 to 18 C atoms as substrate

by microbial transformation with the aid of the Candida tropicalis DSM 3152 mutant with an alkane-degradation defect.

3. Microorganism strain DM 3152 (Candida tropicalis).

**Revendications**

1. Les acides 3-hydroxy-dicarboxyliques de formule

$$HOOC - CH_2 - \underset{\displaystyle |}{\overset{OH}{CH}} - (CH_2)_n - COOH \qquad (I),$$

dans laquelle n est un nombre entier de 9 à 14.

2. Procédé pour la préparation d'acides 3-hydroxy-dicarboxyliques de la formule (I), dans laquelle n est un nombre entier de 8 à 14, caractérisé par le fait qu'on les prépare à partir de n-alcanes comportant de 8 à 14 atomes de carbone en tant que substrat, par transformation microbienne à l'aide du mutant de dégradation défecteuse des alcanes qu'est le Candida tropicalis DSM 3152.

3. La souche de micro-organisme DSM 3152 (Candida tropicalis).